Europäisches Patentamt

**European Patent Office**

**Office européen des brevets**

(19)

(11) Publication number: **0 068 785**
A1

(12)

# EUROPEAN PATENT APPLICATION

(21) Application number: **82303230.5**

(22) Date of filing: **21.06.82**

(51) Int. Cl.³: **C 07 C 11/09**, C 07 C 31/04,
C 07 C 1/20, C 07 C 29/00

(30) Priority: **19.06.81 JP 95701/81**

(43) Date of publication of application: **05.01.83**
**Bulletin 83/1**

(84) Designated Contracting States: **DE FR GB IT NL**

(71) Applicant: **SUMITOMO CHEMICAL COMPANY, LIMITED,
15 Kitahama 5-chome Higashi-ku, Osaka-shi Osaka-fu
(JP)**

(72) Inventor: **Deguchi, Takashi, 15-17, Nishi
Yakura 3-chome, Kustsu Shiga-ken (JP)**
Inventor: **Tokumaru, Tooru, 2200, Oaza Tsurusaki, Oita
Oita-ken (JP)**

(74) Representative: **Geering, Keith Edwin et al, REDDIE &
GROSE 16 Theobalds Road, London WC1X 8PL (GB)**

(54) **Process for the decomposition of methyl-tert-butyl ether.**

(57) A process for recovering isobutylene (B) and methanol (C) by decomposing methyl-tert-butyl ether (MTBE) (A) is provided, which comprises a reaction distillation wherein (a) MTBE is decomposed in the presence of acid catalyst in the liquid phase (1), (b) decomposition product is distilled under reflux in a distillation column (2) to separate isobutylene from the top of the said column and methanol from a side cut effluent and/or effluent from the bottom of the said column. Methanol is preferably separated (3) from the effluent which is then returned to (a) and/or (b).

PROCESS FOR THE DECOMPOSITION
OF METHYL-TERT-BUTYL ETHER

The present invention pertains to a process for recovering isobutylene and methanol from the decomposition of methyl-tert-butyl ether (abbreviated to MTBE hereinafter).

There has long been a need in the petrochemical industry to develop a process for efficient utilization of $C_4$ fractions. Particularly, so called spent BB (abbreviated to SBB hereinafter), which is the residue after the extraction of butadiene, has been used mostly for fuel and rarely as an effective raw material for petrochemistry. There has recently been developed a process wherein isobutylene in SBB is made to react with methanol to separate the former as MTBE which can be employed as one of the components of gasoline. However, the additional profit of utilizing MTBE for fuel is not necessarily large.

On the other hand, a method of direct oxidation of isobutylene to produce methacrylic acid has recently come to be employed industrially and this is an example of the highly efficient use of isobutylene as a raw material in petrochemistry. Isobutylene can be used as a raw material for producing other chemical products such as butyl rubber, isoprene and 4-methyl-2,6-di-tert-butyl phenol.

It is necessary to separate isobutylene from SBB inexpensively if its use as a raw chemical material is to be economical. However, it is impossible to separate the SBB components simply by distillation, because of their close boiling points, and a method has been adopted wherein isobutylene is extracted from SBB with aqueous sulfuric acid and then released; however, this method has many drawbacks such as the need for equipment of expensive material and for expensive waste water treatment.

It is also known to separate isobutylene from SBB by the tert-butanol or MTBE process wherein isobutylene is converted to tert-butanol or MTBE, which is separated from the reaction mixture and then decomposed to recover isobutylene. The MTBE method is preferable in terms of effective utilization of n-butene, i.e. the residue after isobutylene is separated from the MTBE decomposition mixture, because the reaction of isobutylene and methanol proceeds selectively and quantitatively under milder conditions in the presence of ion exchange resin as a catalyst. For the decomposition of MTBE a vapour phase catalytic reaction is well known, but has the drawbacks that heat charge is large because of the high reaction temperature, unfavorable side reactions run to produce by-products such as dimethyl ether, and catalyst life is comparatively short so that frequent catalyst regeneration and/or repacking may be necessary.

The present invention provides an improved process for recovery of isobutylene and methanol by decomposition of MTBE and hence permits more efficient utilization of SBB.

The process of the present invention is characterised by decomposing the MTBE in the liquid phase in the presence of acid catalyst, and distilling the decomposition products under reflux to yield an overhead fraction composed mainly of isobutylene and a side cut and/or bottoms effluent containing methanol.

The reaction distillation process of this invention may be one wherein (a) decomposition of MTBE is conducted in the liquid phase in the presence of acid catalyst, (b) the reaction mixture is heated and led to a distillation column in the vapour phase, (c) the fraction composed mainly of isobutylene is separated from the top of the said distillation column whilst proper reflux is conducted, (d) methanol is separated from a side cut effluent and/or effluent from the bottom of the said distillation column, and (e) the remainder of the said effluent is recycled to the said distillation column and/or the reaction.

The decomposition reaction is conducted in the presence of acid catalyst in the liquid phase. The catalyst employed in this invention may be a mineral acid such as sulfuric acid or phosphoric acid, organic sulfonic acid, or solid acid catalyst such as activated alumina, silica-alumina, metal sulphate or ion exchange resin. Sulfonated ion exchange resin is a preferred catalyst because it does not corrode the equipment used.

Preferred catalyst concentration is 0.1 to 20 wt.% of the reaction mixture. Reaction temperature is suitably 60 - 150$^{o}$C., more preferably 80 - 120$^{o}$C. Reaction rate may be too low below 60$^{o}$C, and catalyst decomposition and undue formation of by-product dimethyl ether tend to occur above 150$^{o}$C. The decomposition reaction in the present invention is generally conducted at the boil, so that the reaction temperature depends upon the reaction pressure. Appropriate reaction pressure is for example 1 - 6 kg/cm$^{2}$.G (Gauge), preferably 2 - 5 kg/cm$^{2}$.G. Reaction solvent is not essential but solvent other than methanol or MTBE may be used, e.g. a hydrocarbon such as iso-octene.

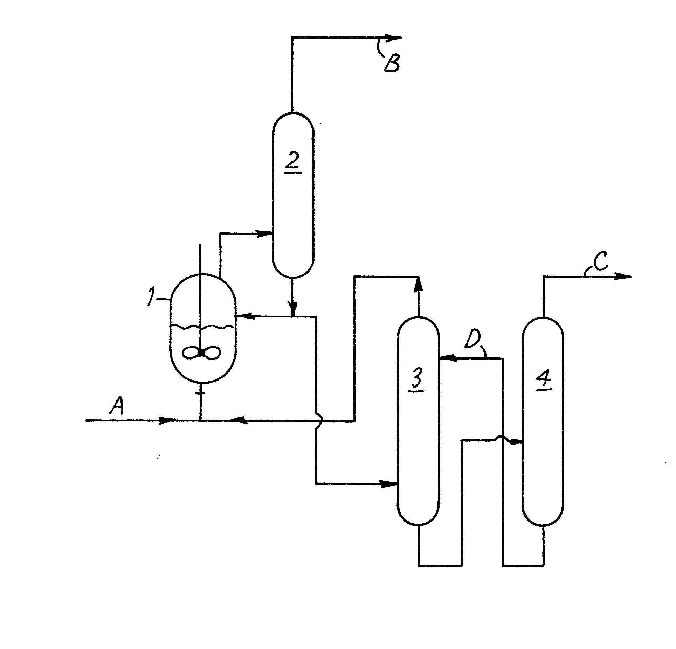

The accompanying drawing is a schematic block diagram of an embodiment of this invention. Referring to the drawing the vapour generated in the reactor 1 is introduced to a distillation column 2 directly connected to reactor 1. Within limits, better results are attained as the number of theoretical plates in said column increases. In practice, at least 3 plates are preferable, but increase beyond 10 plates does not necessarily improve results further. Reflux is conducted at the top zone of the distillation column 2 in the present invention. The higher the reflux ratio employed, the greater the efficiency obtainable but the greater the heat expenditure necessary. The preferred reflux ratio R/F (wherein R and F represent the weights per unit hour of reflux and of feedstock supply respectively) is 1 - 10, more preferably 3 - 6.

Methanol, which is one of the decomposition products of MTBE, is separated from the side cut effluent or the effluent from the bottom of the distillation column 2. For separating methanol from said effluent any conventional methods may be employed; of these, extraction with water is preferred, because methanol forms an azeotropic mixture with MTBE; alternatively the extraction of methanol with water may be conducted after concentration of methanol by distillation. The remainder (mainly MTBE) of said effluent after the separation of methanol can be returned to the distillation column 2 or the reactor 1. The separation of methanol in the side cut effluent or the effluent from the bottom of the distillation column is essential in the present invention. If methanol is taken out together with isobutylene from the top of the distillation column, without separation as aforesaid, the conversion of MTBE is insufficient and much heat expenditure is necessary for the recovery and recycling of unreacted MTBE. The process of this invention, in contrast, can give substantially quantitative conversion of MTBE, and the effluent from the top of the distillation column can be supplied as a starting material, e.g. for oxidation to methacrylic acid, without complicated pre-treatment.

In the drawing, 3 is a column for extracting methanol with water and 4 is a distillation column for the recovery of methanol. The MTBE feedstock of the reaction is supplied via a conduit A, while isobutylene and methanol are recovered via conduits B and C respectively.

The present invention is further illustrated by the following specific non-limiting Example using a system as shown in the drawing.

## EXAMPLE

10 dry wt. parts of strong acid type ion exchange resin (Duolite C-26H type grade from Diamond Schamlock) and 100 wt. parts of MTBE were introduced into a pressure vessel reactor 1 equipped with stirrer and stirred under heating. Distillation column 2 connected directly to reactor 1 is of about 5 theoretical plates. While maintaining a pressure of 3 mg/cm$^2$.G, the MTBE feedstock was supplied to the reactor at a rate of 100 wt. parts per hour and at the same time the effluent from the top of the distillation column is taken off at a rate of 68 wt. parts per hour and the residue (only organic phase) is refluxed. The reflux ratio R/F was about 5. The effluent from the bottom of the distillation column 2 was taken off at a rate of 300 wt. parts per hour and washed with water at a rate of 100 wt. parts per hour in counterflow, and then the effluent was returned to the reactor 1. When the reaction distillation according to the aforesaid conditions reached a steady state, the composition of the effluent from the top of the distillation column was isobutylene 93.2 wt.%, methanol 1.6 wt.%, MTBE 0.46 wt.%, dimethyl ether 0.32 wt.% and water 4.4 wt.%; thus the conversion of MTBE was 99.7% and the by-production ratio of dimethylether was 0.8 mol.%. Methanol was recovered by distillation of the water-washed effluent. The temperature of the liquid reaction mixture was $93^{\circ}$C.

### Comparative Example

The procedure of the above Example was repeated but without extraction of methanol from the bottoms liquid from the distillation column and with effluent being taken off from the top of the distillation column at almost the same rate as that of the MTBE supply. In the steady state the conversion of MTBE was 47.9 % and the by-production ratio of dimethyl ether per MTBE reacted was 2.9 %. The temperature of the liquid reaction mixture was $95^{\circ}$C.

C L A I M S :

1.      A process for the recovery of isobutylene and methanol from methyl-tert-butyl ether (MTBE) characterised by decomposing the MTBE in the liquid phase in the presence of acid catalyst, and distilling the decomposition products under reflux to yield an overhead fraction composed mainly of isobutylene and a side cut and/or bottoms effluent containing methanol.

2.      A process according to claim 1 including separating methanol from the side cut and/or bottoms effluent, and recycling the remainder of said effluent to the decomposition and/or distillation.

3.      A process according to claim 2 characterised in that methanol is separated by water washing of the effluent.

4.      A process according to any preceding claim characterised in that the acid catylist is a sulfonic acid type ion exchange resin.

5.      A process according to any preceding claim characterised in that the reaction pressure is from 1 to 6 kg/cm$^2$.G.

0068785

6.      A process according to any preceding claim characterised in that the reflux wt. ratio is from 1 to 10.


7.      A reaction distillation process for recovering isobutylene and methanol by decomposing methyl-tert-butyl ether (MTBE) wherein (a) decomposition of MTBE is conducted in the liquid phase in the presence of acid catalyst, (b) the reaction mixture is heated and led to a distillation column in the vapour phase, (c) the fraction composed mainly of isobutylene  is separated from the top of the said distillation column and reflux is conducted, (d) methanol is separated from the side cut effluent and/or the effluent from the bottom of the said distillation column, (e) the remainder of said effluents is recycled to the said distillation column and/or the reaction.

## DOCUMENTS CONSIDERED TO BE RELEVANT

| Category | Citation of document with indication, where appropriate, of relevant passages | Relevant to claim |
|---|---|---|
| X | US - A - 4 232 177 (SMITH JR.)<br><br>+ Examples 4,5; column 7, lines 13-38; claim 1; column 5, lines 22-29 +<br><br>-- | 1,4-7 |
| A | DE - B2 - 1 934 422 (SHELL)<br><br>+ Claim 1; column 8, line 45 - column 12, line 65; examples 5-8, especially column 18, lines 14-22 +<br><br>-- | 1,5,7 |
| A | US - A - 3 170 000 (VERDOL)<br><br>+ Example 23, especially table VI +<br><br>-- | 1,3 |
| A | GB - A - 2 025 454 (SNAMPROGETTI)<br><br>+ Example 1, especially table 2; claims 10,12 +<br><br>-- | 1,5 |
| A | US - A - 3 637 889 (WATANABE et al.)<br><br>+ Example 2 +<br><br>---- | |

**CLASSIFICATION OF THE APPLICATION (Int. Cl. 3)**

C 07 C 11/09
C 07 C 31/04
C 07 C 1/20
C 07 C 29/00

**TECHNICAL FIELDS SEARCHED (Int.Cl. 3)**

C 07 C 11/00
C 07 C 31/00
C 07 C 1/00
C 07 C 29/00
C 07 C 7/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| X | The present search report has been drawn up for all claims |
|---|---|

| Place of search | Date of completion of the search | Examiner |
|---|---|---|
| VIENNA | 27-08-1982 | KÖRBER |